# EUROPEAN PATENT SPECIFICATION

(11) **EP 2 214 568 B1**
(45) Date of publication and mention of the grant of the patent: **14.08.2013**
(21) Application number: 08848733.5
(22) Date of filing: 14.11.2008
(51) Int. Cl.: A61B 17/06, A61B 17/00, A61B 17/04

(54) **DEVICE FOR THE MODELLING OR THE LIFTING OF NORMAL OR DISTENDED TISSUE IN HUMAN BEING**
VORRICHTUNG ZUR MODELLIERUNG ODER STRAFFUNG VON NORMALEM ODER AUSGEDEHNTEM GEWEBE BEI MENSCHEN
DISPOSITIF POUR LE MODELAGE OU LE REDRAPAGE D'UN TISSU NORMAL OU DISTENDU CHEZ L'ÊTRE HUMAIN

(30) Priority: 15.11.2007 US 996402 P
(43) Date of publication of application: 11.08.2010
(73) Proprietor: Devonec, Marian, 01700 Miribel (FR)
(72) Inventor: Devonec, Marian, 01700 Miribel (FR)
(74) Representative: Maureau, Philippe
(86) International application number: PCT/EP2008/065584
(87) International publication number: WO 2009/063064

(56) References cited:
- EP-A- 0 464 480
- EP-A- 0 632 999
- WO-A-2005/016176
- WO-A-2007/098535
- US-A- 4 741 330
- US-A1- 2007 142 846
- US-B1- 6 491 714

## Description

The present invention is about the modelling or the lifting of normal or distended soft tissue in human being (including animals too). More precisely, the invention concerns a device, and an assembly comprising this device, to model normal soft tissue or to re-establish the original position of a distended anatomical structure, i.e. by bringing closer two anatomical structures. Thus, it is to be understood by "normal tissue" (e.g. "normal anatomical structure") a tissue which has kept its originally state.

The modelling or the reestablishment of distended anatomical structure can be a medical necessity for some people or they may have an aesthetic motivation. Indeed, the deformation (such that a distension) of a tissue can occur after a local traumatism or can be simply due to aging.
In this technical field, it is well-known to use the technique of open surgery for the modelling or lifting of normal or distended tissue.

The material used in this latter technique consists in a thread mounted on a needle, which is passed through two distant anatomical structures, before the two strands of the thread are tied. The tension of the two strands is adjusted until the distance desired between the two structures is obtained. Furthermore, before the stitches are put in the appropriate place of the two distant anatomical structures, open surgery is required to expose the two anatomical structures, i.e. the mobile tissue to be pulled and the stable tissue used as an anchor.
Thus, this technique requires not only a thread and a needle, but also other tools, which are at least a retractor to expose the operating field, a forceps, a pair of scissors to dissect, and a needle holder to place stitches.

The use of this technique has several disadvantages.

First of all, it is to be noticed that this technique is an invasive technique, which requires anaesthesia adapted to the invasiveness of the operation. As it is well-known, anaesthesia modifies the natural tone of the tissues. Thus, it makes difficult or even impossible to evaluate in real time the anatomical or functional result of the operation of modelling or lifting.

Secondly, this technique of open surgery modifies the normal anatomy by dissection and exposure of tissues, resulting in the correction of an already modified anatomy, i.e. not representative of its original state before surgery.

Lastly, the operation by open surgery is followed by a healing process, which is proportional to the importance of the surgical traumatism. The healing process can deteriorate the immediate satisfactory result acquired after the operation.

It is therefore an object of the present invention to provide a device, an assembly comprising this device and a method for the modelling or the lifting of normal or distended soft tissue in humans, which do not imply the above mentioned drawbacks of the open surgery.
Indeed, the present invention avoids open surgery by using minimally invasive material and percutaneous technique to model or to lift normal or distended soft tissue in humans, which is hereafter referred as "target tissue".

The two part form of appended claim 1 is based on the disclosure of EP-A-632999 or US-A-4741330. The present invention consists in a device as in appended claim 1.

It is to be noticed that the scope of the present invention covers:
- not only a sustaining means which is already attached to the thread, but also
- a sustaining means which can be delivered separately from the thread and is assembled at the proximal end of the said thread just before the positioning of the said device.

The present invention concerns an assembly for modelling or lifting a target tissue, which comprises:
- at least one device as above described,
- a means of insertion adapted to contain the anchoring means of said device in a compressed state and to deliver the said anchoring means in the anchoring tissue,
- means adapted to successively deliver and clamp the sustaining means of the device below the target tissue and cut the thread below the said sustaining means.

Preferably, the latter mentioned means, which is conceived to successively deliver, clamp the sustaining means of the device below the target tissue and cut the thread below the said sustaining means, may consist in a clip applier.

Furthermore, the present invention concerns an assembly for modelling or lifting a target tissue as described above and which further comprises a dilation means allowing the dilation of the point of penetration of the means of insertion and of the route giving access to the target tissue.

The assembly may moreover comprise a prosthetic material.

In preferred embodiments of the invention, the prosthetic material may consist in a mesh or a tape or a tube or a plain rod.

Preferably, the means of insertion may consist in a needle.

It is to be noticed that the different elements of the assembly according to the present invention may be miniaturized, biocompatible, non-resorbable and they may be metallic or non-metallic.

In an embodiment of the invention, the anchoring means may consist in a hook equipped with an radially outwardly expandable member.
Furthermore, the sustaining means of the device according to the present invention may consist in a washer in association with a clip.

According to the present invention, the clip may have the shape of a "U" shape or a perforated round plate or a sphere with a channel or a harpoon or a pins clip.

According to the present invention, the washer is a mesh.

Preferably, the washer may be made of poly-propylene or metallic compound.

In preferred embodiments of the invention, the thread may be metallic or made of poly-propylene or another synthetic bio-compatible compound.

According to the present invention, the proximal end of the thread may comprise at least one notch.

The present invention is now described with references to annexed drawings herein:
- Fig 1a shows a device according to the present invention,
- Fig 1b shows a device without a sustaining means,
- Fig 2a and 2b show two different embodiments of the anchoring means inserted in a means of insertion according to the present invention,
- Fig 3 shows a device according to the present invention
- Fig 4a to 4d show four different embodiments of a clip according to the present invention,,
- Fig 5 shows a washer according to the present invention,
- Fig 6a to 6e show schematically an assembly according to the present invention to model or lift a target tissue,
- Fig 7a and 7b show two different embodiments of the invention in which a plurality of devices according to the invention are attached to the same clip,
- Fig 8a and 8b show the positioning of a plurality of devices according to the invention,
- Fig 9a to 9c show three different embodiments of a prosthetic material according to the present invention,
- Fig 10a and 10b show two different embodiments of a device with a prosthetic material according to the invention,
- Fig 11a to 11c show schematically the positioning of a device according to the invention in breast to solve the problem of breast ptosis in females,
- Fig 12a to 12f show schematically the positioning of a device according to different embodiments to solve the problem of incontinence in females.

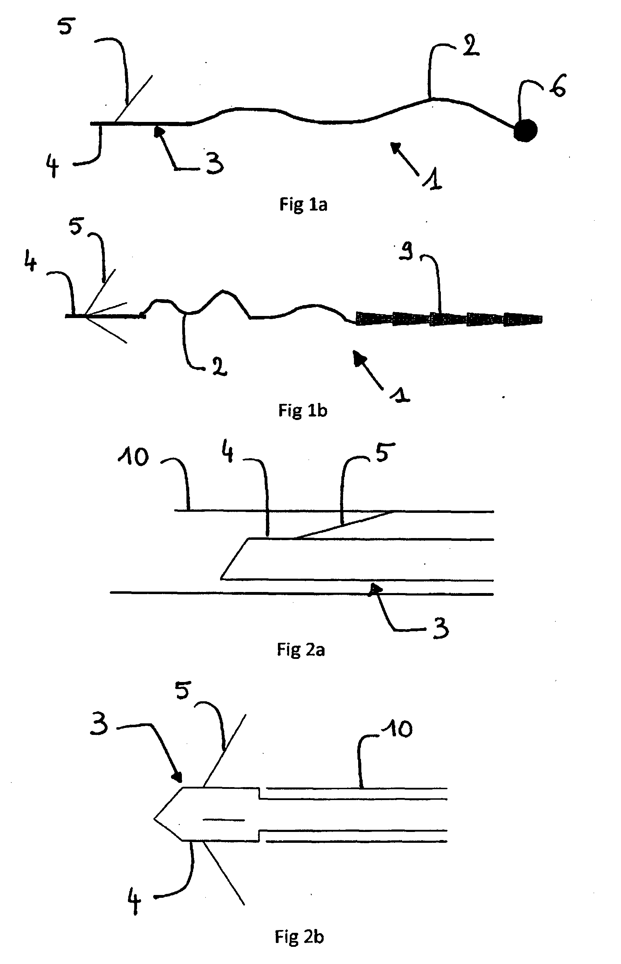

As shown in Fig 1a, the device 1 comprises a thread 2 having at its distal end an anchoring means 3 consisting in a hook 4 equipped with an expandable radially outwardly member 5 and said thread 2 having at its proximal end a sustaining means 6. The hook 4 may be sealed to the thread 2 or built in with the thread 2.

The anchoring means 3 may be metallic or non-metallic.
The anchoring means 3 is flexible and expandable from a compressed to an expanded state.
As shown in Fig 1b, the hook 4 may comprise a plurality of expandable members 5. In an embodiment of the invention, the expandable member is a branch.
Furthermore, the expandable member (for example a branch) may be elastic and have a shape memory.

As shown in Fig 2a, the anchoring means 3 has a size allowing:
- its insertion inside the lumen of a means of insertion 10, which may be a needle,
- its gliding in a compressed state along the lumen of the said means of insertion 10.
The anchoring means 3 is pushed by the thread 2 in the direction of the tip of the means of insertion 10 and it spreads by itself as soon as it is released outside of the tip of the means of insertion 10.

In a preferred embodiment of the invention, the means of insertion 10, which may consist in a needle, has a diameter inferior or equal to 1 mm (i.e. 19.5 G).

The section of the means of insertion 10 is adapted to the dimensions of the device 1. The dimensions and the features of the said device 1 depend on the resistance and forces necessary for the lifting of the target tissue.

The length of the means of insertion 10 is adapted to the distance between the point of puncture of the surface layer of the skin and the anchoring tissue.
The means of insertion 10 may have a straight or a curved shape which is adapted according to:
- the local anatomy,
- the planed route between the point of puncture of the surface layer and the relative positions of the target tissue, the anchoring tissue, and furthermore
- the anatomical structures to avoid.

As shown in Fig 2a, the hook 4 may be a shaft which extends longitudinally with a constant section. Furthermore, as shown in Fig 2a, the means of insertion 10 (such a needle) may have a bevel at its distal end so that it facilitates the puncture through the skin and the tissues. The means of insertion 10 houses the thread 2 and the hook 4.
In another embodiment of the invention, the means of insertion 10 has not a bevel at its distal end.

In another embodiment of the invention and as shown in Fig 2b, the hook 4 may consist in a shaft having two parts with different sections. In this embodiment, the distal tip of the shaft is outside the means of insertion 10 and the diameter of the tip of the shaft is equal to the external diameter of the means of insertion 10. It corresponds to the first section of the said shaft. The base of the shaft which is inside the means of insertion 10 (i.e. the second section) has a diameter smaller than the one of the lumen of the said means of insertion 10. The hook 4 is driven and pushed by the right angle tip of the means of insertion 10. In this embodiment shown in Fig 2b, the tip of the hook 4 punctures the skin and the tissues. So, in this embodiment, it is not necessary that the means of insertion 10 has a bevel. Furthermore, in this embodiment shown in Fig 2b, the expandable members 5 flatten during tissue puncture and spread when pulling on the thread 2.
More precisely, in this embodiment, when the tip of the hook 4 is outside the means of insertion 10, the branches of the hook 4 are in the spread position. As soon as the superficial layer has been punctured by the tip of the hook 4, the branches of the hook 4 flatten as they come in contact with the superficial layer. They remain flat until the tip of the hook 4 has reached the anchoring tissue. As this point, the thread does not need to be pushed, since the hook is already outside the means of insertion 10. The elasticity and shape memory of the branches of the hook 4 allow them to expand in the anchoring tissue as soon as the thread 2 is pulled.

It is to be noticed that the means of insertion 10 with the smallest possible section offers the following advantages:
- an anaesthesia free puncture: the pain generated by the puncture necessary to deliver the anaesthesia is equivalent to the pain generated by the puncture delivering the treatment. Thus, anaesthesia can be avoided.
- an easy progression through the tissues facilitating the guidance of the means of insertion 10.
- a tactile feed-back of the resistance of the tissues during progression with manual guidance,
- a precise route,
- a minimal damage to the tissue punctured,
- multiple punctures during a single session.

The thread 2 may be conceived to be stiff enough for pushing and gliding the anchoring means 3 through the lumen of the means of insertion 10. Furthermore, the thread 2 may have a necessary flexibility to minimize the local traumatism to the tissues crossed by the said device 1.

The device 1 is conceived to be adapted to the necessary resistance to support the lifting of the target tissue and the physiological forces exerted in the area where it is implanted. It is important to minimize the volume of the device 1 in order to reduce the tissue reaction to the implant.

The thread 2 may be metallic or non-metallic.

In a preferred embodiment of the invention, the thread 2 is metallic, because it offers the following advantages: a small section is allowed with the required stiffness, flexibility, resistance and tolerance of the said thread 2.
The metal can be chosen for its mechanical properties but also for its biological properties, like good biocompatibility, or even for its therapeutic properties. The metal may deliver particles to the tissue surrounding the thread 2, along the whole length or only a given segment of the thread. The particles can be delivered spontaneously from the metal itself or after incorporation or addition to the mental.

In another embodiment of the invention, the thread 2 may be made of non-metallic compound such that poly-propylene or another bio-compatible compound.

In another embodiment of the invention, the thread 2 may further incorporate a therapeutic compound along the whole length or of a given segment of the said thread 2.

In a preferred embodiment of the invention, the thread 2 is a mono-filament structure. Indeed, the mono-filament structure provides stiffness to the thread 2.

In an embodiment of the invention, the surface of the thread 2 is flat. It provides the following avantages:
- the gliding of tissues along the thread 2,
- the adjustment of the tension of the thread 2 between the target and the anchoring tissues, before the fixation of the new position of the said target tissue,
- the finding of the new position for the target tissue in accordance with the aesthetic or functional goal of the procedure.

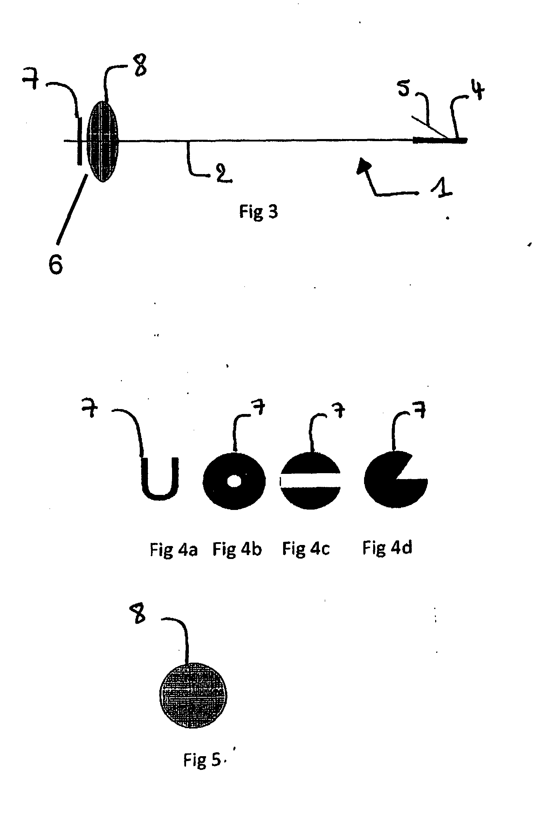

The figure 3 shows a device 1 according to the present invention.
As shown in this figure, the sustaining means 6 consists in the association of a washer 8 with a clip 7.

The thread 2 passes through the washer 8.The clip 7:
- is secured to the proximal end of the thread 2,
- blocks the washer 8 which is pressed against the target tissue in a tense position.
Furthermore, in the case of a thread 2 having a monofilament structure, it allows an easy gliding of the washer 8 and clip 7 along the said thread 2.

It is important that the thread 2 is stiff enough to be introduced by its proximal tip through the washer 8 which consists in a mesh without the need of another tool such a surgery needle.

As shown, in Fig 4a to 4d, the clip 7 according to the present invention may have different shapes:
- an "U" shape (Fig 4a),
- a round plate with a hole, preferably in its centre ( Fig 4b), i.e. a disc,
- a sphere with a channel ( Fig 4c), or
- a notch ( Fig 4d), like a harpoon,
- a pins clip, like for a piercing jewel.

The shapes of "U", disc, or sphere are particularly advantageous, because it allows an easy gliding along the device 1.

The shape of 'U' is particularly preferred.

Furthermore, in a disclosed embodiment which is shown in Fig 1b, the proximal end of the thread 2 may comprise at least one notch 9 which extends longitudinally.
In this embodiment, the clip 7 may have the shape of a round plate with a hole, whose size is precisely adjusted so that the round plate automatically blocks at the level of one of the said notches 9.

The clip 7 may be metallic or non- metallic. In a preferred embodiment of the invention, the clip 7 is metallic and that facilitates the miniaturization of the said clip 7.
In an embodiment of the invention, the clip 7 may be made of a malleable compound such that titanium.

In an embodiment of the invention, the clip 7 has a section smaller than the one of the washer 8 under which it is placed.

The washer 8 is
- a mesh made of poly-propylene (or other synthetic biocompatible compound) or metallic compound, as shown in Fig 5.

The mesh has pores having a diameter such that it allows the passage of the thread 2.
More precisely, it is preferably to conciliate the following parameters:
- the section of the thread 2, and
   - the pore of the mesh,
so that it allows the passage of the thread 2 through one of the pores of the said mesh of the washer 8 without previous dilation of the said pore.

Furthermore it may be advantageous that the thread 2 is stiff and flexible so that it facilitates its introduction through the said mesh without the need of a tool such a needle.

It is to be noticed that the section of the washer 8 is adapted both to the resistance of the target tissue and to the strength of the traction force. The washer prevents the section of the target tissue by the clip 7, especially when the clip has a "U" shape and transformed in a "I" shape after crushing by the means delivering, clamping the sustaining means 6. Furthermore, the section of the washer 8 may be minimal to reduce the size of the access port necessary to place the washer 8 under the target tissue and to reduce the local reaction of the tissue to the prosthetic material which may be further implanted.

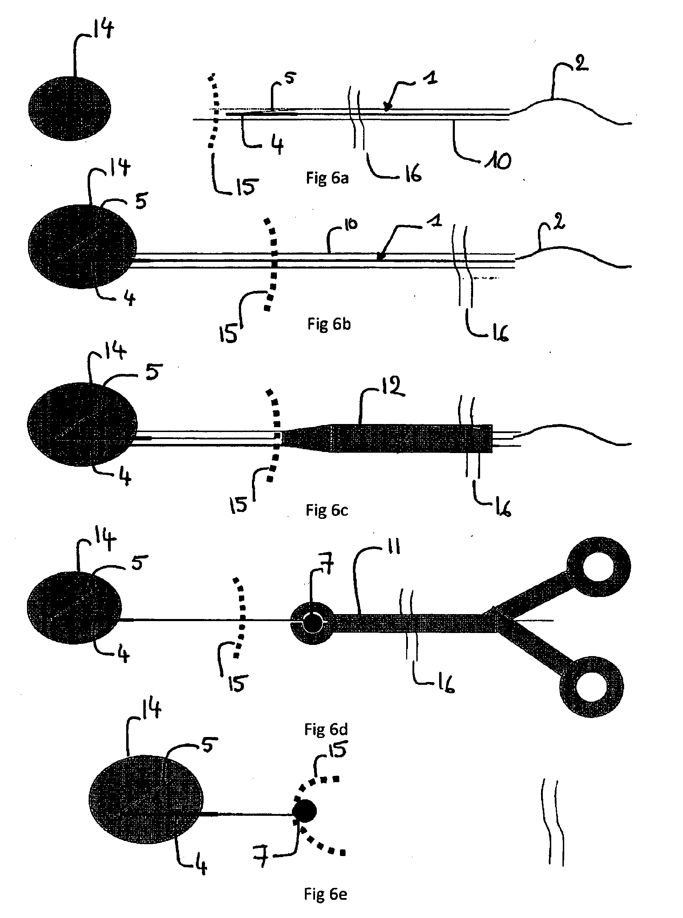

It is disclosed an embodiment in which the proximal end of the thread 2 comprises at least one notch 9 as shown in Fig 1b, the perforated washer 8 allows the lifting of the target tissue and may play the role of a clip. The washer may have a hole with a diameter slightly inferior to the maximum diameter of the segment of the As shown, in Fig 6d, the assembly according to the present invention comprises a means 11 conceived to have successively the following functions:
- to deliver and to clamp the sustaining means 6 of the device 1 below the target tissue, and
- to cut the thread 2 below the said sustaining means 6.

This means 11 may be a clip applier.

Thus, the said means 11 pushes the sustaining means 6 along the thread 2, more precisely, in the case of a sustaining means 6 consisting in the association of a clip 7 and a washer 8, the said means 11 presses the clip 7 against the washer 8 supporting the target tissue and secures it in the desired position.

In a preferred embodiment of the invention, the means 11 has a tip whose size is approximately the size of the washer 8 and the clip 7 to allow its insertion through the skin and tissues without any complementary dilation of the latter.

According to the present invention, the assembly may optionally comprise a dilation means 12 allowing the dilation of the point of penetration of the means 11 and of the route giving access to the target tissue.

In a preferred embodiment of the invention, the dilation means 12 may consist in a dilation tube. More preferably, the section of said dilation tube is equal to the one of the washer 8 and the one of the clip 7.

In an embodiment of the invention, the dilation means 12 may have a conical distal tip.

Furthermore, said dilation means 12 may have a lumen whose section allows:
- the position of said dilation means 12 surrounding the means of insertion 10, and
- an easy gliding along the said means of insertion 10.

The length of said dilation means 12 may be sufficient to reach the target tissue.

The dilation means 12 may further comprise at least one longitudinal blade, which may be incorporated to the cone in the case of a dilation means having a conical distal tip. Thus, according to this embodiment, dilation is performed more easily. It widens the route to the target tissue.

The figures 6a to 6e show the assembly according to the present invention.

More precisely, Fig 6a shows the device 1 comprising a thread 2 having at its distal end a hook 4 equipped with an expandable member 5 which is in a compressed state because said device 1 is inserted into the means of insertion 10. As shown in this figure 6a, the means of insertion 10, which contains the device 1, has punctured the skin 16 and has reached the target tissue 15. It is to be noticed that the progression may be manually guided or, if needed, instrumentally guided by an imaging means like ultra-sonography or fluoroscopy.

Thus, the tip of the means of insertion 10 follows the desired route, passes first through the target tissue 15 and finishes its course inside the anchoring tissue 14. A tactile resistance may be perceived by the operator, when the means of insertion 10 punctures the anchoring tissue 14.

As shown in Fig 6b, when the tip of the means of insertion 10 has reached the anchoring tissue 14, the hook 4 is pushed and pulled by the thread 2 without the need of any other tool. The anchoring means 3 is definitely fixed in the anchoring tissue 14 by a slight pulling of the thread 2. In other words, the thread 2 is pushed so that the hook 4 penetrates and spreads inside the anchoring tissue 14. The fixation of the anchoring means 3 (in the present embodiment a hook 4) is obtained by a slight pulling on the thread 2. The resistance felt means efficacious anchoring.

As shown in Fig 6c, a dilation means 12 consisting in a dilation tube is positioned by surrounding the means of insertion 10 and pushed along the said means of insertion 10 to reach the target tissue 15. Thus, the point of penetration of the means of insertion 10 is dilated and the route between the skin 16 and the target tissue 15 is dilated too.

Then, the dilation means 12 and the means of insertion 10 are removed.

As shown in Fig 6d, the means 11 conceived to deliver and to clamp the sustaining means 6 is introduced in the above mentioned dilated route and surrounds the device 1. As shown in this figure 6d, the means 11 may consist in a clip applier.

As not shown, the proximal tip of the thread 2 is passed through the sustaining means 6 which consists in the association of a washer 8 and a clip 7. The thread 2 is then pulled, while the washer 8 and the clip 7 are pushed and glided forward along the thread 2.

As not shown, once the washer 8 is in contact with the target tissue 15, the necessary tension appropriate to the target tissue 15 is adjusted. At this point the clip 7 is secured on the thread 2 and the said thread 2 is cut with the means 10 below the clip 7 as it is shown in Fig 6e. The means 11 is removed.
It could be understood that the three successive functions of delivering, clamping the sustaining means 6 and cutting the thread 2 may be accomplished separately with at least two specific means.

For instance, it could be possible to have two different means:
- the first one delivering and clamping the sustaining means 6, and
- the second one cutting the said thread 2.

Thus, a method using a device 1 according to the present invention may be carried out to model or to lift a target tissue.

Thus, an assembly as above described may be carried out to model or to lift a target tissue.

Hereafter, other embodiments according to the present invention are described.

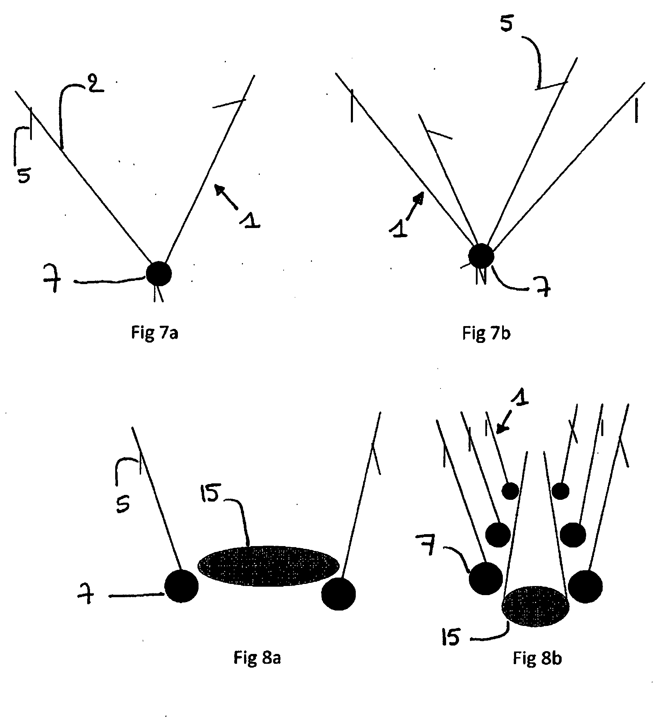

The device 1 may comprise a plurality of threads 2.
As shown in Fig 7a, the device 1 comprises two threads 2 having at their distal end a hook 4, said threads 2 being attached to the same clip 7 at their proximal ends.
As shown in Fig 7b, the device 1 comprises four threads 2 having at their distal end a hook 4, said threads 2 being attached at their proximal ends to the same clip 7.

The choice of these embodiments shown in Fig 7a and 7b are made according to the anatomical and physiological features of the target tissue 15. Furthermore, embodiments of the invention as shown in Fig 8a and 8b may be carried out.
As shown respectively in Fig 8a and 8b, the disposition of the devices 1 may be symmetrical on both sides of the target tissue 15 which may be an orifice (Fig 8a) or of a natural channel (Fig 8b).
The disposition of the devices 1 may be asymmetrical too.

The disposition and the number of required devices 1 according to the present invention are adapted until the anatomic or functional modelling is judged satisfactory.

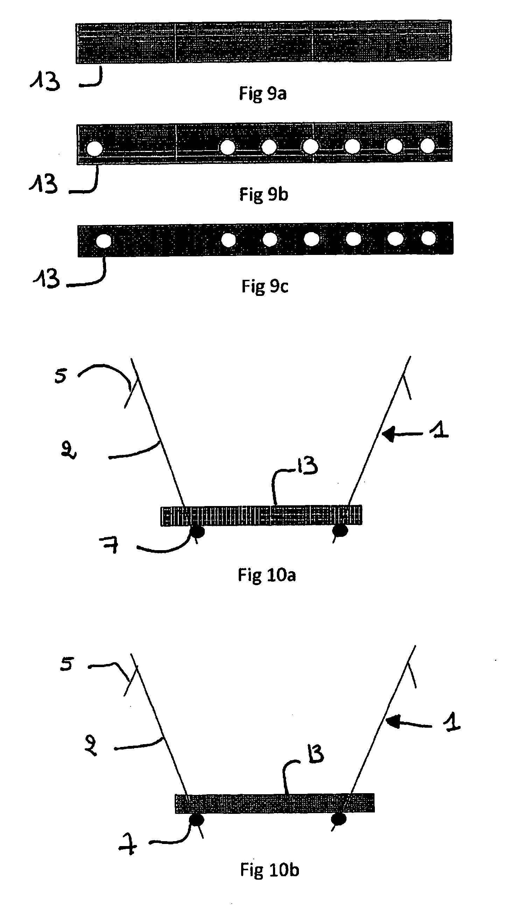

The assembly according to the present invention may further comprise a prosthetic material 13 which is shown in Fig 9a to 9c.

The prosthetic material 13 may consist in a mesh, a tape, a tube or a plain rod,
The prosthetic material 13 may be metallic or non-metallic.
The prosthetic material 13 may be made of poly-propylene or any other synthetic bio-compatible compound.
The procedure to position the device 1 according to the present invention with this further prosthetic material 13 is the same as the one described above and as it was shown in Fig 6a to 6e. Thus, as above mentioned, the necessary tension appropriate to the target tissue 15 is adjusted.

In the case of a mesh or a tape, the width and length are adapted to the target tissue 15 to be supported.
For example, for stress urinary incontinence, the mesh may have a width of 5 mm and a length of 30 mm.

As shown in Fig 9a, the prosthetic material 13 may be a mesh.

In another embodiment of the invention, the prosthetic material 13 may be a mesh with holes.

In an embodiment of the invention, the disposition of the holes is asymmetrical. As shown in Fig 9b, one side of the mesh has a single hole, whereas the opposite side has several holes positioned at equidistant intervals, in order to adjust the dimension of the prosthetic material 13 to each individual anatomy. The length is cut and tailored according to each situation.

As shown in Fig 9c, the prosthetic material 13 may be a tube comprising holes. The diameter and length are adapted to the target tissue to be supported. For example, for stress urinary incontinence: 2 mm diameter and 30mm long. The holes are disposed at each extremity of the said tube. The thread 2 may pass through a channel along the axis of the tube. The length of the tube is cut according to the local anatomy.

Fig 10a shows an embodiment of the invention in which the prosthetic material 13 is a mesh and two devices 1, disposed at its ends, pass through the said prosthetic material 13.

Fig 10b shows another embodiment of the invention in which the prosthetic material 13 is a tube and two devices 1 disposed at its ends pass through this prosthetic material 13.

The present invention offers the following advantages:

Firstly, the elements of the assembly according to the present invention may be miniaturized, which translate into minimal local trauma and into precise placement of the device 1. Furthermore, several devices 1 may be positioned in the same area as it was shown in Figures 8a and 8b.

Secondly, the assembly according to the present invention to model or to lift target tissue requires an anaesthesia free lifting technique. Indeed, the means of insertion 10, such a thin needle used for the positioning of the device 1 does not require previously a local anaesthesia. Only when several devices 1 have to be placed in a same area accessible to a single anaesthesia puncture, local infiltration with a local anaesthetic drug may be justified.

Furthermore, it is important to notice that the absence of anaesthesia combined with the minimal traumatism allows the evaluation of the result during the insertion of the device 1 and an eventual adjustment.

The insertion and the positioning of the device 1 is non-traumatic and an immediate result due to the absence or at most a minor healing process is obtained. It is important to notice the absence of resting recommendations usually needed during healing process. Furthermore, there is no modification of the immediate result by the healing process and the scar.

The positioning of the device 1 does not require an incision. It consists in a percutaneous technique. Furthermore, it may be performed on an original state or non-modified anatomy.

As it has already been mentioned, the device 1 according to the present invention may be used alone or in combination with a complementary prosthetic material 13, i.e. a mesh or a tape or a tube placed below the target tissue or organ. In this case, a dissection is necessary to position the prosthetic material 13.

The present invention offers adaptability concerning the target tissue. Indeed, the clip 7 of the sustaining means 6 may be inserted inside, just below the target tissue 15, or may be left outside the target tissue 15. More precisely, the clip 7 may be fixed below the target tissue 15, when it cannot be left outside, either because of the depth of the target tissue 15, or because of cosmetic or biological reasons.

It is possible to position the clip 7 outside the target tissue 15. The procedure may be slightly different when the target tissue 15 is superficial or, when the technique is used for a non-cosmetic application.
The clip 7 may be left outside the point of penetration of the means of insertion 10, directly in contact with the target tissue 15, like a piercing jewel. In this case the thread 2 may have an antiseptic property per se or due to the incorporation to the thread 2 of a therapeutic compound released to prevent infection.

The outside position of the sustaining means 6 and eventually complementary prosthetic material 13 allow performing a second procedure to adjust the lifting or to remove the thread or threads.

The inside positioning of the sustaining means 6 may be indicated for a definitive implantation.

The outside positioning of the sustaining means 6 clip or of the clip plus supporting material may be indicated for a temporary implantation. The positioning may be used as a reversible test:
- when the result is satisfactory, the device 1 may be implanted inside, during a second procedure, which will consist to remove the sustaining means 6 placed outside and to complete the regular procedure. A new sustaining means 6 will be placed inside, below the target tissue;
- when the result is not satisfactory the mounting can be either adjusted or removed:
   o adjustment is possible from outside: after removal of the sustaining means 6, the thread 2 is pulled and a new sustaining means 6 is secured in a new position, above or below the previous position to give respectively more or less tension to the lifting. In order to be able to proceed to such adjustment, a few millimetre length of thread 2 must be left below the sustaining means 6 at the end of the initial procedure,
   o removal may be obtained by vigorous pulling. For example, in an embodiment of the invention, in which the anchoring means 3 consist in a hook 4 equipped with branches, the said branches of the hook 4 fold backwards in the direction of the tip of the shaft. The thread 2 is removed with minimal tissue traumatism. Removal can be performed without any incision or operation.

It is important to notice that the positioning of the device 1 is adjustable due to:
- the possibility to lift more or less by pushing more or less the sustaining means 6 along the thread 2 before the final fixing,
- the possibility to lift more or less by cutting more or less the length of the complementary prosthetic means 13 due to the presence of several holes at one extremity of the tape or tube as it has been above mentioned.

The device 1 according to the present invention may represent a way to deliver to a target tissue a therapy via particles released by the thread 2 naturally or after previous incorporation of said particles to the thread 2.

The device 1 according to the present invention may allow the modelling or the lifting of human or animal tissues.

The assembly according to the present invention may allow the modelling or the lifting of human or animal tissues.

The device 1 and the assembly according to the present invention may be used in several applications. Hereafter, several applications are mentioned. They do not represent limitations of the present invention.

The device 1 according to the present invention may be used for the treatment of anal incontinence.
The assembly according to the present invention may be used for the treatment of anal incontinence.

The device 1 may be used to carry out a face lifting of distended tissue due to aging. Thus, it may concern face lifting for the repair of facial palsy.
It is possible to carry out the face lifting by using the assembly according to the present invention.

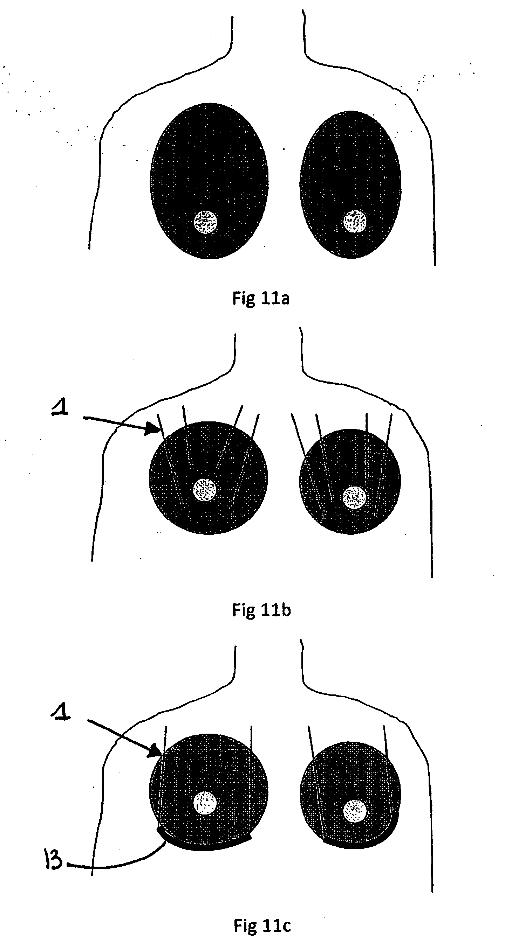

As shown in Fig 11a to 11c, the device 1 according to the present invention may be used for breast lifting. It may concern breast ptosis, which has to be corrected.
Fig 11 a represents the breasts before the positioning of the device 1 according to the present invention.
As shown in Fig 11b, a plurality of devices 1 may be positioned, eventually symmetrically, in each mammary gland.
Furthermore, as shown in Fig 1c, breast ptosis as evoked may also be corrected by two threads 2 supporting a tape placed in the groove below the breast. In an embodiment of the invention, the positioning results in a permanent sub-cutaneous bra. Sustaining means 6 such clips 7 and complementary material (such prosthetic material 13) are left inside, when used for cosmetic repair.

Furthermore, the present invention may have uses in the technical field of urology.
More precisely, it may concern stress urinary incontinence in females or males.

Thus, the device 1 according to the present invention may be used to treat stress urinary incontinence.

The assembly according to the present invention may be used to treat stress urinary incontinence.

For this use, precisely to treat stress urinary incontinence in females, several embodiments of the present invention may be suitable.

Hereafter, some of these embodiments are disclosed:

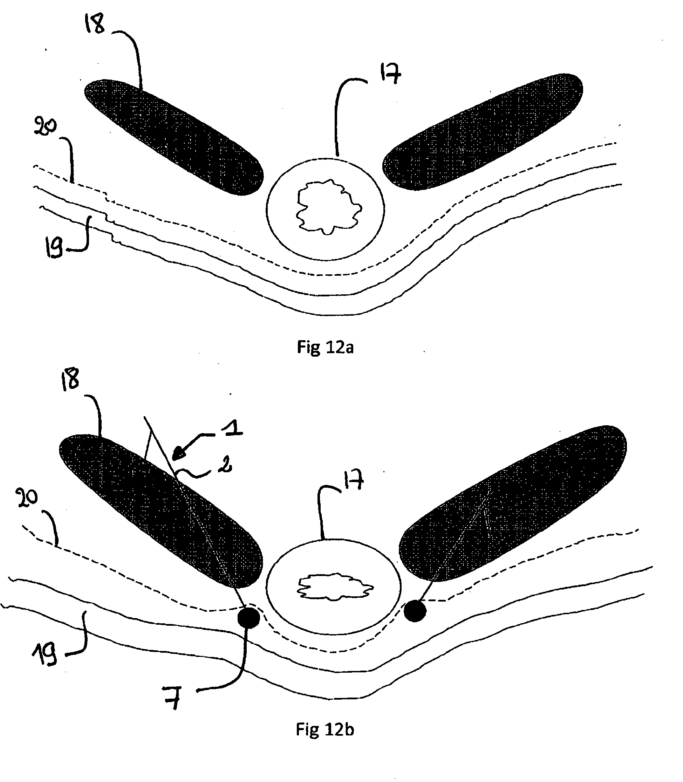

Fig 12a shows the urethra 17 and the levator ani muscle 18 before the positioning of the device 1.

Fig 12b shows two devices 1, more precisely two threads 2 with clip 7 as sustaining means 6 inside the vaginal wall 19, which lift superficial fascia 20.

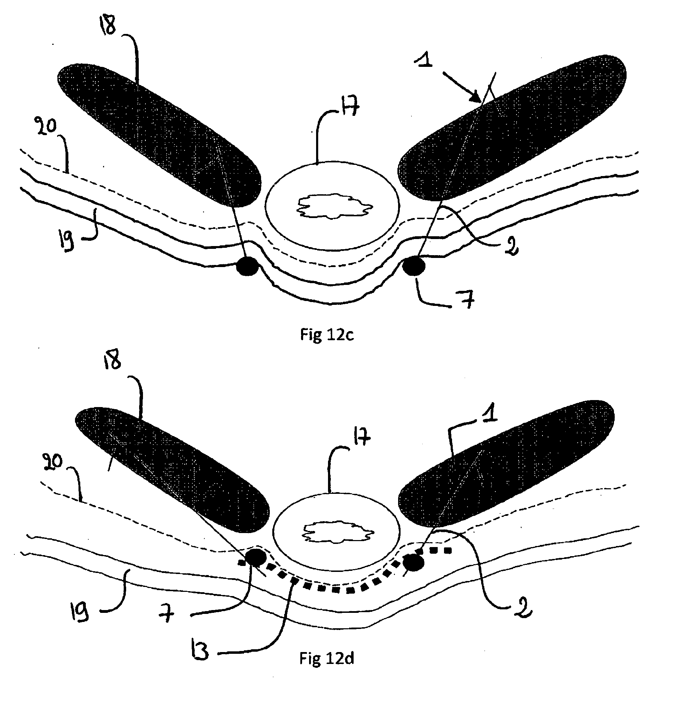

Fig 12c shows two devices 1, more precisely two threads 2 with clip 7 outside the vaginal wall 19, which lift the said vaginal wall 19.

Fig 12d shows two devices 1, more precisely two threads 2 with clip 7 inside the vaginal wall 19, and a prosthetic material 13 consisting in a tape, which lift the superficial fascia 20.
Another embodiment would be two devices 1, more precisely two threads 2 with clip 7 outside the vaginal wall 19, and a prosthetic material 13, such a tape, which lift the vaginal wall 19.

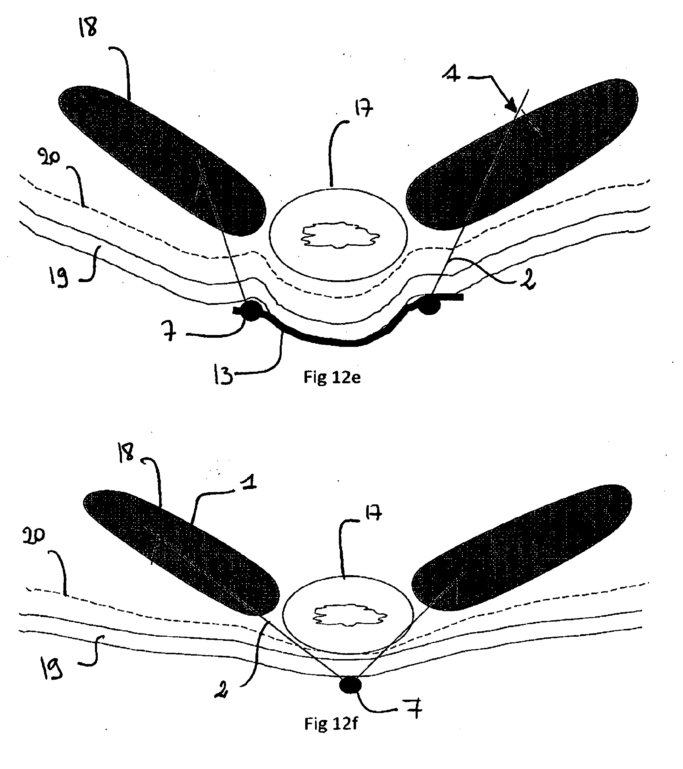

Fig 12e shows two devices 1, more precisely two threads 2 with clip 7 outside the vaginal wall 19, and a prosthetic material 13 consisting in a tube, which lift the said vaginal wall 19.

Another embodiment would be two devices 1, more precisely two threads 2 with clip 7 inside the vaginal wall 19, and a prosthetic material 13 consisting in a tube, which lift the superficial fascia 20.

Fig 12f shows two devices 1, more precisely two threads 2 which are attached to the same clip 7. The clip 7 is outside the vaginal wall 19. Each device 1 lifts the vaginal wall 19. The lifting has a V shape.

Another embodiment would be two devices 1, more precisely two threads 2 which are attached to the same clip 7. The clip 7 is inside the vaginal wall 19. Each device 1 lifts the superficial fascia 20.

According to these two latter embodiments, the cure of stress urinary incontinence in females may be simply performed after one puncture on each side of the female urethra 17 during the positioning of the device 1 according to the present invention. Furthermore, the direct access to the clip 7 (in the embodiment in which the clip 7 is outside the vaginal wall) allows the differed adjustment of the mounting if necessary, or its removal by a vigorous instant pulling of the said clip 7.

The outside positioning of the tape, tube and of the clips on the luminal side of the vaginal wall allows checking reversibly the potential cure of urinary incontinence by this technique. In case of success, the tape, tube and clips may be implanted inside during a second procedure. In case of failure, the threads 2 may be removed simply by pulling without any operation.

Furthermore, other embodiments in which the site of anchoring may differ are possible too. As examples, anchoring may be performed in the levator ani muscle 18 for a U shape lifting (as shown in Fig 12b to 12f), or in the obturator intemus muscle for a wide open U shape lifting.

It is to be noticed that concerning the treatment of stress urinary incontinence in males, the same principles as disclosed above for females, may be applied. The device 1 is preferentially placed inside the skin of the perineum using an inside positioning. Anchoring is performed in the levator ani for a U shape lifting, or in the corpus cavernosum for an open U shape lifting.

## Claims

1. Device (1) for modelling or lifting a target tissue comprising at least one thread (2), said thread (2) having:
- at its distal end a radially outwardly expandable anchoring means (3) configured to anchor to a stable anchoring tissue (14), and
- at its proximal end a sustaining means (6) configured to lift a mobile target tissue (15),
so that the target tissue (15) is linked with the anchoring tissue (14) thanks to said device (1), **characterized in that** the sustaining means (6) consists in a mesh washer (8) in association with a clip (7) which are configured to be pushed and slid along the thread (2).

2. Device (1) according to claim 1 wherein the anchoring means (3) consists in a hook (4) equipped with an expandable radially outwardly member (5).

3. Device (1) according to claim 1 or 2 wherein the clip (7) has the shape of a "U" shape or a perforated round plate or a sphere with a channel or a harpoon or a pins clip.

4. Device (1) according to anyone of the preceding claims 3 wherein the mesh washer (8) is made of poly-propylene or metallic compound.

5. Device (1) according to anyone of the preceding claims wherein the thread (2) is metallic or made of poly-propylene or another synthetic bio-compatible compound.

6. Device (1) according to any one of the preceding claims wherein the proximal end of the thread (2) comprises at least one notch (9).

7. Assembly for modelling or lifting a target tissue (15), which comprises:
- at least one device (1) according to any one of the preceding claims,
- a means of insertion (10) adapted to contain the anchoring means (3) of said device (1) in a compressed state and to deliver the said anchoring means (3) in the anchoring tissue (14),
- means (11) adapted to successively deliver, and clamp the sustaining means (6) of the device (1) below the target tissue (15) and to cut the thread (2) below the said sustaining means (6).

8. Assembly according to claim 7 wherein said assembly further comprise a dilation means (12) allowing the dilation of the point of penetration of the means of insertion (10) and of the route giving access to the target tissue (15).

9. Assembly according to claim 7 or 8 wherein said assembly further comprises a prosthetic material (13).

10. Assembly according to claim 9 wherein the prosthetic material (13) consists in a mesh or a tape or a tube or a plain rod.

11. Assembly according to anyone of claims 7 to 10 wherein the means of insertion (10) consists in a needle.

12. Assembly according to anyone of claims 7 to 10 wherein the delivery means (11) is a clip applier.

## Patentansprüche

1. Vorrichtung (1) zum Modellieren oder Anheben eines Zielgewebes, umfassend zumindest einen Faden (2), wobei der Faden (2) aufweist:
- an seinem distalen Ende ein radial nach außen erweiterbares Verankerungsmittel (3), das so konfiguriert ist, dass es an einem stabilen Verankerungsgewebe (14) verankert, und
- an seinem proximalen Ende ein Stützmittel (6), das so konfiguriert ist, dass es ein bewegliches Zielgewebe (15) anhebt,
so dass das Zielgewebe (15) durch die Vorrichtung (1) mit dem Verankerungsgewebe (14) verbunden ist, **dadurch gekennzeichnet, dass** das Stützmittel (6) aus einer Netzunterlegscheibe (8) in Verbindung mit einer Klemme (7) besteht, die so konfiguriert sind, dass sie entlang des Fadens (2) geschoben werden und gleiten.

2. Vorrichtung (1) nach Anspruch 1, wobei das Verankerungsmittel (3) aus einem Haken (4) besteht, der mit einem radial nach außen erweiterbaren Element (5) versehen ist.

3. Vorrichtung (1) nach Anspruch 1 oder 2, wobei die Klemme (7) die Form einer U-Form oder einer perforierten runden Platte oder einer Kugel mit einem Kanal oder einer Harpune oder einer Steckklammer hat.

4. Vorrichtung (1) nach einem der vorstehenden Ansprüche 3, wobei die Netzunterlegscheibe (8) aus Polypropylen oder einer metallischen Verbindung hergestellt ist.

5. Vorrichtung (1) nach einem der vorstehenden Ansprüche, wobei der Faden (2) metallisch oder aus Polypropylen oder einer anderen synthetischen biologisch kompatiblen Verbindung hergestellt ist.

6. Vorrichtung (1) nach einem der vorstehenden Ansprüche, wobei das proximale Ende des Fadens (2) zumindest eine Kerbe (9) umfasst.

7. Anordnung zum Modellieren oder Anheben eines Zielgewebes (15), die umfasst:
- zumindest eine Vorrichtung (1) nach einem der vorstehenden Ansprüche,
- ein Einsetzmittel (10), das so ausgelegt ist, dass es das Verankerungsmittel (3) der Vorrichtung (1) in einem zusammengedrückten Zustand enthält und das Verankerungsmittel (3) im Verankerungsgewebe (14) zuführt,
- ein Mittel (11), das so ausgelegt ist, dass es das Stützmittel (6) der Vorrichtung (1) unterhalb des Zielgewebes (15) sukzessive zuführt und klemmt und den Faden (2) unterhalb des Stützmittels (6) schneidet.

8. Anordnung nach Anspruch 7, wobei die Anordnung ferner ein Erweiterungsmittel (12) umfasst, das die Erweiterung des Penetrationspunktes des Einsetzmittels (10) und des Weges, der Zugang zum Zielgewebe (15) bereitstellt, ermöglicht.

9. Anordnung nach Anspruch 7 oder 8, wobei die Anordnung darüber hinaus ein prothetisches Material (13) umfasst.

10. Anordnung nach Anspruch 9, wobei das prothetische Material (13) aus einem Netz oder einem Band oder einem Rohr oder einer glatten Stange besteht.

11. Anordnung nach einem der Ansprüche 7 bis 10, wobei das Einsetzmittel (10) aus einer Nadel besteht.

12. Anordnung nach einem der Ansprüche 7 bis 10, wobei das Zuführmittel (11) ein Clip-Applikator ist.

## Revendications

1. Dispositif (1) pour modeler ou lever un tissu cible comprenant au moins un fil (2), ledit fil (2) ayant :
- à son extrémité distale un moyen d'ancrage expansible radialement vers l'extérieur (3) configuré pour ancrage à un tissu d'ancrage stable (14), et
- à son extrémité proximale un moyen de soutien (6) configuré pour lever un tissu cible mobile (15),
de sorte que le tissu cible (15) soit lié avec le tissu d'ancrage (14) grâce audit dispositif (1), **caractérisé en ce que** le moyen de soutien (6) est constitué d'une rondelle de grille (8) en association avec une attache (7) qui sont configurées pour être poussées et glissées le long du fil (2).

2. Dispositif (1) selon la revendication 1 dans lequel le moyen d'ancrage (3) est constitué d'un crochet (4) équipé d'un composant expansible radialement vers l'extérieur (5).

3. Dispositif (1) selon la revendication 1 ou 2 dans lequel l'attache (7) a la forme d'un « U » ou d'une plaque ronde perforée ou d'une sphère avec un canal ou un harpon ou une attache à broches.

4. Dispositif (1) selon l'une quelconque des revendications précédentes 3 dans lequel la rondelle de grille (8) est constituée de polypropylène ou d'un composé métallique.

5. Dispositif (1) selon l'une quelconque des revendications précédentes dans lequel le fil (2) est métallique ou constitué de polypropylène ou d'un autre composé synthétique biocompatible.

6. Dispositif (1) selon l'une quelconque des revendications précédentes dans lequel l'extrémité proximale du fil (2) comprend au moins une encoche (9).

7. Ensemble pour modeler ou lever un tissu cible (15), qui comprend :
- au moins un dispositif (1) selon l'une quelconque des revendications précédentes,
- un moyen d'insertion (10) adapté pour contenir le moyen d'ancrage (3) dudit dispositif (1) dans un état comprimé et pour délivrer ledit moyen d'ancrage (3) dans le tissu d'ancrage (14),
- un moyen (11) adapté pour délivrer et serrer avec succès le moyen de soutien (6) du dispositif (1) au-dessous du tissu cible (15) et pour couper le fil (2) au-dessous dudit moyen de soutien (6).

8. Ensemble selon la revendication 7 dans lequel ledit ensemble comprend en outre un moyen de dilatation (12) permettant la dilatation du point de pénétration du moyen d'insertion (10) et de la voie permettant d'accéder au tissu cible (15).

9. Ensemble selon la revendication 7 ou 8, ledit ensemble comprenant en outre un matériau prosthétique (13).

10. Ensemble selon la revendication 9, le matériau prosthétique (13) étant constitué d'une grille ou une bande ou un tube ou une tige plate.

11. Ensemble selon l'une quelconque des revendications 7 à 10, le moyen d'insertion (10) étant constitué d'une aiguille.

12. Ensemble selon l'une quelconque des revendications 7 à 10, le moyen de délivrance (11) étant un applicateur d'attache.
